# NEUE EUROPÄISCHE PATENTSCHRIFT

(11) **EP 0 625 997 B2**
(45) Veröffentlichungstag und Bekanntmachung des Hinweises auf die Entscheidung über den Einspruch: **27.12.2000**
(45) Hinweis auf die Patenterteilung: 07.05.1997
(21) Anmeldenummer: 93903795.8
(22) Anmeldetag: 08.02.1993
(51) Int. Cl.: C08G 18/18, C08G 18/20

(54) **AMINOCARBONAT-VERBINDUNGEN UND DEREN ANWENDUNG ALS KATALYSATOREN**
AMINOCARBONATE COMPOUNDS AND THEIR USE AS CATALYSTS
COMPOSES AMINOCARBONATE ET LEUR UTILISATION COMME CATALYSEURS

(30) Priorität: 11.02.1992 DE 4203908
(43) Veröffentlichungstag der Anmeldung: 30.11.1994
(73) Patentinhaber: AIR PRODUCTS AND CHEMICALS, INC., Allentown, PA 18195-1501 (US)
(72) Erfinder: WAGNER, Arwed, D-50937 Köln (DE); DIBLITZ, Klaus, D-22869 Schnefeld (DE); HOELL, Detlef, D-47443 Moers (DE)
(74) Vertreter: Kador & Partner
(86) Internationale Anmeldenummer: DE9300117
(87) Internationale Veröffentlichungsnummer: WO9316124

(56) Entgegenhaltungen:
- EP-A- 0 088 377
- EP-A- 0 389 098
- FR-A- 2 319 421
- US-A- 3 703 520
- Week 3982, 24. August 1982 Derwent Publications Ltd., London, GB; AN 82-82860E
- Week 8449, 30. Oktober 1984 Derwent Publications Ltd., London, GB; AN 84-304984
- F.G. Willeboordse et al., "Kinetics and Catalysis of Urethane Foam Reactions" in "Journal of Cellular Plastics", January 1965, pp. 76-84

## Beschreibung

Die Erfindung betrifft Aminocarbonat-Verbindungen sowie deren Anwendung als Katalysatoren zur Herstellung von Urethan- und/oder Harnstoff-Polymeren.

Katalysatoren zur Herstellung von Polyurethanen sind bekannt (J.H. Saunders und K.C. Frisch, Polyurethanes Chemistry and Technology, 1962 S. 73 ff). Es sind organische, metallorganische und anorganische Verbindungen. Aus der Gruppe der organischen Verbindungen sind es insbesondere tertiäre Amine wie z.B. Bis(dimethylaminoethyl) ether (US 3 400 157), Aminoorthoester (US 3 786 029) und β,β'-Dimorpholinodiethylester (DE 2 138 403). Beispiele für Metallkatalysatoren sind Sn(II)/Sn(IV)-Salze oder Fe(III)-Salze (DE 3 938 203 A1).

Die derzeit eingesetzten Katalysatoren zeigen jedoch eine Vielzahl von Nachteilen. Viele Amine, wie Bis(dimethylaminoethyl)ester, besitzen einen äußerst unangenehmen Geruch, der sowohl bei der Herstellung von Polyurethanen als auch für die Verarbeitung der mit diesen Katalysatoren hergestellten Polyurethanwerkstoffen von Nachteil ist. Es ist bis heute auch nicht gelungen, eindeutig von der chemischen Struktur einer Verbindung auf deren Geruch und den Eigenschaften als Katalysator für die Herstellung von Polyurethanen zu schließen.

Ein weiterer Gesichtspunkt zur Einstufung eines Katalysators ist die Lage des Gleichgewichts seiner Aktivität gegenüber der Isocyanat/Alkohol-Reaktion sowie der Isocyanat/Wasser-Reaktion. Vergleicht man Aminkatalysatoren mit ähnlichem chemischen Aufbau wie etwa Bis(dimethylaminoethyl)ether und Dimethylaminopropyldimethylaminoethyl-ether, so bewirkt schon eine Verlängerung um lediglich eine Methylengruppe eine deutliche Verminderung der Aktivität und vor allem eine starke Verschiebung der katalytischen Beeinflussung der Isocyanat-Wasser Reaktion in Richtung auf die Isocyanat/Alkohol-Reaktion. Aus einem starken treibaktiven Katalysator wird ein mittelaktiver Gelierkatalysator (N. Malwitz et al, Proceedings of the 30th Annual Polyurethane Technical/Marketing Conference, October 15-17, 1986, S. 338-353).

Des weiteren sind im Stand der Technik Untersuchungen zur Kinetik der Alkohol/Isocyanat bzw. Wasser/Isocyanat Reaktionen sowie der katalytischen Aktivität verschiedener tertiäter Amine in Polyurethanreaktionen bekannt (F.G. Willeboordse et al., Journal of CELLULAR PLASTICS, 1965, S. 76-84).

Zur Verminderung des Geruchs von Aminkatalysatoren hat man daher Aminverbindungen mit hohen Molekulargewichten und damit verbunden niedrigen Dampfdrücken verwendet. Solche Verbindungen erfordern jedoch aufgrund ihrer geringen Beweglichkeit und der dadurch bedingten geringen Aktivität hohe Einsatzkonzentrationen.

Zur Verminderung der Geruchsproblematik ist es weiterhin Stand der Technik, Aminokatalysatoren mit Substituenten zu verwenden, welche gegenüber Isocyanaten reaktive Wasserstoffatome besitzen. Beispiele hierfür sind Dimethylethanolamin und Dimethylaminopropylamin. In JP-A-59 191 743 sind die Umsetzungsprodukte von Polyamin mit Carbonaten als Polyurethan-Katalysatoren beschrieben. Ein großer Nachteil dieser Technik ist das Verbleiben der Aminokatalysatoren im Polyurethan. Hierdurch kann bekanntermaßen die Rückspaltung der Urethan- oder Harnstoffgruppen katalysiert werden, was zu einer Verschlechterung der Hydrolyse- und Alterungsbeständigkeit führt.

Der Erfindung liegt die Aufgabe zugrunde, unter Vermeidung oder Minderung der oben genannten Nachteile neue Verbindungen herzustellen, die als Katalysatoren zur Herstellung von Polyurethanen und/oder Polyharnstoffen geeignet sind.

Erfindungsgemäß erfolgt die Lösung der Aufgabe durch die Bereitstellung von Aminocarbonat-Verbindungen der allgemeinen Formel I 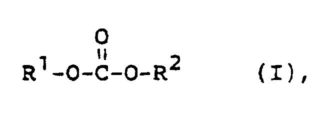 worin R¹ und R², die gleich oder verschieden sind, eine tertiäre Aminogruppe sind, die ein Rest der allgemeinen Formel II 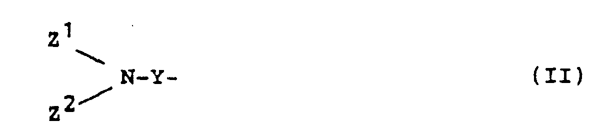 worin Z¹ und Z² zusammen einen Morpholin- oder Piperazin-Rest der allgemeinen Formel III bzw. IV bilden 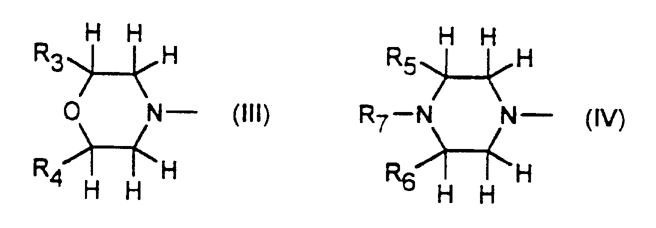 worin die Reste R³ und R⁴ und die Reste R⁵ und R⁶ gleich oder verschieden sind und jeweils Wasserstoff und/oder Alkylreste mit 1 bis 2 Kohlenstoffatomen, und R⁷ Wasserstoff oder einen Alkylrest mit 1 bis 2 Kohlenstoftatome bedeuten, und Y ein unverzweigtes oder verzweigtes Alkyllen mit 2 bis 10 Kohlenstoffatomen bedeutet.

Die erfindungsgemäßen Verbindungen weisen als Katalysatoren überraschende Eigenschaften auf:
- sie besitzen eine ausreichend hohe Aktivität
- sie sind geruchsarm, und
- sie lassen sich aus wohlfeilen Ausgangsverbindungen darstellen.

Die erfindungsgemäßen Katalysatoren zeigen zwar eine etwas geringere Aktivität als strukturverwandte bekannte Verbindungen, die grundsätzliche Charakteristik bleibt jedoch voll erhalten:

Der erfindungsgemäße Katalysator Dimorpholinoethylcarbonat (I) beeinflußt ebenfalls wie β,β'-Dimorpholinodiethylether nur die Isocyanat/Wasser-Reaktion. Vorteilhaft im Sinne der Erfindung ist dabei, daß der erfindungsgemäße Katalysator I nahezu geruchsfrei ist.

Bei den erfindungsgemäßen Verbindungen der allgemeinen Formel I 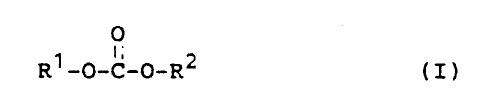 stehen R¹ und R² für gleiche oder verschiedene Reste, die eine tertiäre Aminogruppe der allgemeinen Formel II 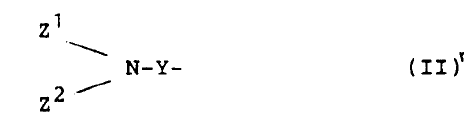 worin Y bevorzugt für ein Alkylen mit 2 bis 4 C-Atomen steht.

Z¹ und Z² bilden zusammen ein Morpholin- oder Piperazinderivat der allgemeinen Formel III bzw. IV. 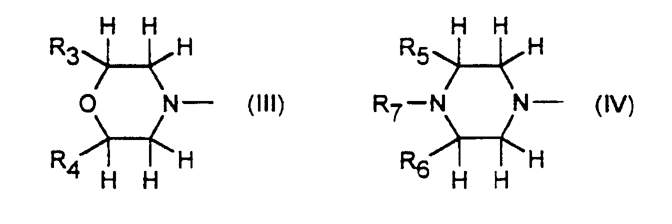 Hierbei bedeuten die Reste R³ bis R⁷ vorzugsweise H-Atome und/oder Methyl.

Die erfindungsgemäßen Verbindungen sind durch die Umsetzung von Aminoalkoholen mit Alkylcarbonaten erhältlich.

Beispiele für Aminoalkohole als geeignete Ausgangssubstanzen sind Hydroxymethylmorpholin, Hydroxyethylmorpholin, Hydroxypropylmorpholin, Hydroxybutylmorpholin, 2-Morpholinylethan-1-ol, 2-(3,5-dimethylmorpholinyl)-ethan-1-ol, 2-Piperazinylethan-1-ol, 2-(1-N-methylpiperazinyl)-ethan-1-ol, 2-(1-N-methyl-3,5-dimethylpiperazinyl)-ethan-1-ol, Hydroxyethoxyethylmorpholin, Hydroxyethoxyethylpiperazin, 1-(1-N-methylpiperazinyl)-3-oxa-5-hydroxypentan, sowie Verbindungen der allgemeinen Formeln V und VI 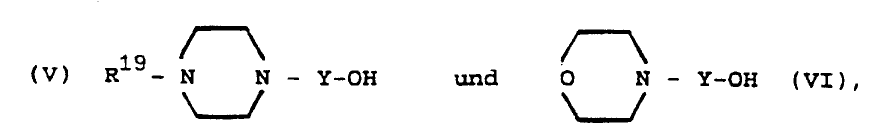 wobei R¹⁹ und Y die oben angegebene Bedeutung besitzen.

Die Alkylcarbonate besitzen bevorzugt einen Alkylrest mit 1 bis 3 C-Atomen. Zur Beschleunigung der Reaktion sind Lewis-Basen geeignet, die entweder Metalle vorzugsweise aus der I und II Hauptgruppe des Periodensystems, Hydroxyverbindungen dieser Metalle oder auch tertiäre Amine darstellen.

Die erfindungsgemäßen Verbindungen eignen sich zur Herstellung von massiven oder zelligen Polyurethanen, wobei die erfindungsgemäßen Katalysatoren allein oder in Kombination mit für die Herstellung von Polyurethanen geeigneten handelsüblichen Katalysatoren eingesetzt werden können. Handelsübliche Katalysatoren können aus der Gruppe der tertiären Amine, Carbonsäuresalze, Phosphorverbindungen und Metallverbindungen stammen.

Als Beispiele für handelsübliche Katalysatoren werden die folgenden Aminkatalysatoren genannt:
Triethylendiamin, Bis(dimethylaminoethyl)ether, Dimethylcyclohexylamin, Dimethylbenzylamin, Dimethylethanolamin, N-Methylmorpholin, N-Ethylmorpholin, Dimorpholinodiethylether, Tetramethylhexamethylendiamin, 2-Methyl-2-azanorbornan, 2-(Hydroxyethoxyethyl)-2-azanorbornan, 2-(2-Dimethylaminoethoxy)-ethanol, 3-Dimethylaminopropyl-diisopropanolamin, Bis(3-dimethylaminopropyl)-isopropanolamin und 2-Dimethylaminoethyl-3-dimethylaminopropylether.

Als Beispiele für handelsübliche Metallkatalysatoren sind werden die folgenden genannt:
Metallsalze, vorzugsweise Zinn, einer Carbonsäure und gemischte Alkyl- und Carbonsäurederivate eines Metalls. So können z.B. Dibutylzinndilaurat, Dibutylzinndiacetat, Diethylzinndiacetat, Zinndioktoat sowie Gemische davon eingesetzt werden.

Weiter ist der Zusatz eines Schaumstabilisators möglich, etwa aus der Klasse der Silane oder Siloxane (US 3 194 773).

Zur Herstellung geschäumter Polyurethane mit den erfindungsgemäßen Verbindungen als Katalysatoren können Polyisocyanate eingesetzt werden. Beispielhaft genannt seien hier Hexamethylendiisocyanat, Phenylendiisocyanat, Toluylendiisocyanat, Isophorondiisocyanat, Naphtylendiisocyanat und 4,4'-Dipenylmethandiisocyanat. Besonders geeignet sind 2,4-Toluylendiisocyanat oder 2,6-Toluylendiisocyanat sowie Mischungen daraus. Weitere geeignete Polyisocyanate sind kommerziell erhältliche Mischungen, bekannt als "crude-MDI", welche in etwa 60% des 4,4'-Diphenylmethandiisocyanats und weitere Isomere oder analoge höhermolekulare Polyisocyanate enthalten. Besonders geeignet sind ebenfalls Mischungen aus Toluylendiisocyanat und 4,4'-Diphenylmethandiisocyanat sowie den als "crude-MDI" bekannten Polyisocyanaten. Ebenfalls geeignet sind "Prepolymere" der oben genannten Polyisocyanate, bestehend aus den Umsetzungsprodukten von Polyisocyanaten und Polyether-oder Polyesterpolyolen.

Die mit den Polyisocyanaten reaktionsfähige Polyolkomponente kann ein Polyesterpolyol oder ein Polyetherpolyol sein. Geeignete Polyole sind Polyalkylen- oder Polyesterpolyole. Besonders geeignete Polyalkylenpolyole umfassen Polyalkylenoxidpolymere, wie etwa Polyethylenoxid-und Polypropylenoxidpolymere sowie gemischt polymerisierte Polyetylen- und Polypropylenoxidpolymere. Startverbindungen für solche Polyalkylenpolyole sind beispielsweise Ethylenglycol, Propylenglycol, 1,3-Butandiol, 1,4-Butandiol, 1,6-Hexandiol, Neopentylglycol, Diethylenglycol, Dipropylenglycol, Pentaerithritol, Glycerin, Diglycerin, Trimethylolpropan, Cyclohexandiol, Sucrose und Saccharose.

Geeignete Polyesterpolyole umfassen die Reaktionsprodukte aus Dicarbonsäuren mit einem Überschuß an Diolen, z.B. Adipinsäure mit Ethylenglycol oder Butandiol oder den Reaktionsprodukten aus Lactonen mit einem Überschuß an einem Diol, z.B. Caprolacton und Propylenglycol.

Die folgenden Beispiele dienen der Erläuterung der Erfindung

### Beispiel 1

### Dimorpholinoethylcarbonat (I)

Es wurden 329,0 g (2,5 Mol) Hydroxyethylmorpholin in einem wie oben ausgerüsteten Reaktionskolben vorgelegt. Darin wurden 6,0 g (0,1 Mol) Kaliumhydroxid gelöst und anschließend 35 ml Cyclohexan zudosiert. Die Lösung wurde auf 90°C erhitzt. Innerhalb von 10 Minuten wurden 121,5 g (1,35 Mol) Dimethylcarbonat tropfenweise zugegeben. Nach 36 h Reaktionsdauer wurde der Ansatz filtriert und anschließend im Ölpumpenvakuum (0,1 Torr) destilliert. Nach Abzug von drei Leichtsiederfraktionen erhielt man das gewünschte Produkt bei einer Kopftemperatur von 160-163°C. Die Ausbeute betrug 55% der Theorie. Die Reindestillation (0.3 Torr) ergab I in 98,9 %iger Reinheit (Kopftemperatur 181°C, Siedepunkt von I: 181°C, Ausbeute 54 % der Theorie).

### Beispiel 2

Anwendung der nach Beispiel 1 erhaltenen Verbindung I als Katalysatoren zusammen mit einem Zinnkatalysator bei der Herstellung von Polyurethan-Weichschaum.

Die Herstellung des Polyurethan-Weichschaums erfolgte nach dem Handmischverfahren. Dabei wurde zuerst die A-Komponente, sie enthielt ein geeignetes Polyol, einen Schaumstabilisator, einen Zinnkatalysator sowie den erfindungsgemäßen Aminkatalysator sowie Wasser als Treibmittel, mit einem Hochleistungsrüher bei 1000 U/min 50 s gerührt. Anschließend wurde als B-Komponente die benötigte Menge eines geeigneten Polyisocyanates hinzugegeben, 7 s bei 2500 U/min gerührt und das schaumfähige Gemisch in eine würfelförmige Form (Kantenlänge 27 cm) gegeben. Mittels einer mit einer Ultraschallmeßsonde gekoppelten Meßdatenerfassungssystem wurden die Steigkuryen aufgezeichnet. Aus den so erhaltenen Steigkurven wurden die Cremezeiten sowie die Steigzeiten und Steighöhen ermittelt.

Zur Verschäumung wurde die folgende Rezeptur verwendet:

| | |
|---|---|
| Polyol (1) | 100,0 g |
| Isocyanat (2) | 59,0 g |
| Wasser | 5,0 g |
| Stabilisator (3) | 1,0 g |
| Zinnkatalysator (4) | 0,2 g |
| Aminkatalysator | siehe Tabelle 1 |
| Index (Verhältnis Isocyanat/Polyol) | 106 |

| | |
|---|---|
| (1) verzweigtes Polyol mit einer OH-Zahl von 45-50 und einer mittleren Molmasse von 3400 g/mol | |
| (2) Toluylendiisocyanat mit 80 % 2,4-Isomerer und 20% 2,6-Isomerer | |
| (3) Polyethersiloxan | |
| (4) Zinndioktoat | |

**Tabelle 1**

| Kennwerte der Verschäumung | | | | | |
|---|---|---|---|---|---|
| Katalysator | Menge [pphp] | Cremezeit [s] | Steigzeit [s] | Dichte [kg/m³] | Temp. [°C] |
| DMDEE (1) | 0,2 | 16 | 104 | 26,5 | 22 |
| I (erf.gem.) | 0,2 | 17 | 112 | 25,2 | 22 |
| CD (2) | 0,1 | 7 | 59 | 23,6 | 30 |
| Mischung A | 0,2 | 7 | 73 | 19,7 | 30 |
| Mischung A | 0,2 | 18 | 103 | 21,1 | 20 |
| TD 100 (3) | 0,2 | 16 | 74 | 20,3 | 21 |
| CD (2) | 0,2 | 12 | 58 | 20,0 | 21 |

| | | | | | |
|---|---|---|---|---|---|
| (1) DMDEE = Dimorpholinodiethylether | | | | | |
| (2) CD = Bis(dimethylaminoethyl)ether | | | | | |
| (3) TD 100= Triethylendiamin | | | | | |
| Mischung A: 12 Gew.% TD 100, 19 Gew.% Dimethylethanolamin, 14 Gew.% CD, 55 Gew.% Dipropylenglycol | | | | | |

### Beispiel 3

Anwendung der nach Beispiel 1 erhaltenen Verbindung I als Katalysator bei der Herstellung von Polyurethan-Weichschaum.

Die Herstellung des Polyurethan-Weichschaums erfolgte nach dem Handmischverfahren. Dabei wurde zuerst die A-Komponente, sie enthielt ein geeignetes Polyol, einen Schaumstabilisator, den Aminkatalysator sowie Wasser als Treibmittel, mit dem Hochleistungsrüher bei 1000 U/min 30 s gerührt. Anschließend wurde als B-Komponente die benötigte Menge eines geeigneten Polyisocyanates hinzugegeben, 5 s bei 2000 U/min gerührt und sodann das schaumfähige Gemisch in eine würfelförmige Form (Kantenlänge 27 cm) gegeben. Mittels des mit einer Ultraschallmeßsonde gekoppelten Meßdatenerfassungssystems wurden die Steigkurven aufgezeichnet. Aus den so erhaltenen Steigkurven wurden die Cremezeiten sowie die Steigzeiten und Steighöhen ermittelt.

Es wurde die folgende Formulierung eingesetzt:

| | |
|---|---|
| Polyol (1) | 100,0 g |
| Isocyanat (2) | 20,8 g |
| Isocyanat (3) | 24,8 g |
| Stabilisator (4) | 1,0 g |
| Wasser | 3,7 g |
| Amincokatalysator (5) | 0,4 g |
| Aminkatalysator | siehe Tabelle 2 |

| | |
|---|---|
| (1) Polyesterpolyol, mittleres Molekulargewicht 2400 g/mol, OH-Zahl 57-63 | |
| (2) Toluylendiisocyanat, 80 % 2,4 Isomer, 20 % 2,6-Isomer | |
| (3) Toluylendiisocyanat, 65 % 2,4-Isomer, 35 % 2,6-Isomer | |
| (4) Polyethersiloxan | |
| (5) Dimethylbenzylamin | |

**Tabelle 2**

| Kennwerte der Verschäumung | | | | |
|---|---|---|---|---|
| Katalysator | Menge [pphp] | Cremezeit [s] | Steigzeit [s] | Dicke [kg/m³] |
| N-Methylmorpholin | 3,0 | 15 | 82 | 33,3 |
| Dimethylpiperazin | 1,0 | 15 | 67 | 28,2 |
| I (erf.gem.) | 4,0 | 19 | 110 | 33,5 |

### Beispiel 4

Anwendung der nach Beispiel 1 erhaltenen Verbindung als Katalysator bei der Herstellung von Polyurethan-Hartschaum.

Die Herstellung des Polyurethan-Hartschaums erfolgte nach dem Handmischverfahren. Dabei wurde zuerst die A-Komponente, sie enthielt ein geeignetes Polyol, einen Schaumstabilisator, Wasser, sowie den Aminkatalysator, mit dem Hochleistungsrüher bei 1000 U/min 50 s gerührt. Danach wurde die benötigte Menge eines physikalischen Treibmittels hinzugegeben und 10 s bei 1000 U/min gerührt. Anschließend wurde als B-Komponente die benötigte Menge eines geeigneten Polyisocyanates hinzugegeben, 7 s bei 2500 U/min gerührt und das schaumfähige Gemisch in eine würfelförmige Form (Kantenlänge 27 cm) gegeben. Mittels des mit einer Ultraschallmeßsonde gekoppelten Meßdatenerfassungssystems wurden die Steigkurven aufgezeichnet. Aus den so erhaltenen Steigkurven wurden die Cremezeiten sowie die Steigzeiten und Steighöhen ermittelt.

Zur Verschäumung wurde die folgende Rezeptur verwendet:

| | |
|---|---|
| Polyol (1) | 100,0 g |
| Isocyanat (2) | 126,0 g |
| Wasser | 2,0 g |
| Stabilisator (3) | 1,5 g |
| Treibmittel (4) | 31,0 g |
| Aminkatalysator | siehe Tabelle 3 |
| Index | 105 |

| | |
|---|---|
| (2) Isocyanat: Mischung aus Isomeren des Diphenylmethandiisocyanates mit einem NCO-Gehalt von 7,5 mmol/g | |
| (3) Polyethersiloxan | |
| (3) Polyethersiloxan | |
| (4) Frigen R 11 (CCl₃F) | |

**Tabelle 3**

| Kennwerte der Verschäumung | | | | |
|---|---|---|---|---|
| Katalysator | Menge [pphp] | Cremezeit [s] | Steigzeit [s] | Dichte [kg/m³] |
| Mischung C (erf.gem.) | 8 | 25 | 128 | 24.1 |
| Mischung D (erf.gem.) | 4 | 10 | 220 | 23,5 |
| Mischung E (erf.gem.) | 4 | 34 | 321 | 23,2 |
| Mischung F (erf.gem.) | 8 | 30 | 137 | 24,3 |
| Mischung G (erf.gem.) | 4 | 10 | 253 | 24,6 |
| Mischung H (erf.gem.) | 4 | 38 | 414 | 24,2 |
| I (erf. gem.) | 4 | 180 | 620 | 42,6 |
| TD 100 (1) | 2 | 32 | 159 | 24,5 |
| CD (2) | 2 | 10 | 256 | 24,5 |
| DMCHA (3) | 2 | 43 | 394 | 24,0 |
| unkatakysiert | - | >400 | nicht meßbar | - |

| | | | | |
|---|---|---|---|---|
| (1) TD 100 = Triethylendiamin | | | | |
| (2) CD = Bis(dimethylaminoethyl)ether | | | | |
| (3) DMCHA = Dimethylcyclohexylamin | | | | |

### Beispiel 5

Anwendung der nach Beispiel 1 erhaltenen Verbindung als Katalysator zur Herstellung von massiven Polyurethanen.

Die Herstellung der Polyurethangießharzmasse erfolgte durch Vermengen der A-Komponente, bestehend aus einem geeigneten Polyol, Zuschlagstoffen wie Schwerspat, einem Zeolith zur Wasserbindung und dem Katalysator, mit der B-Komponente, bestehend aus einem geeigneten Polyisocyanat. Zur Charakterisierung wurde die Topfzeit, die Entformzeit sowie die Temperatur der Polyurethangießharzmasse beim Erreichen der Topfzeit bestimmt.

Zur Herstellung der Polyurethangießharzmasse diente die folgende Formulierung:

| | |
|---|---|
| Polyol (1) | 100,0 g |
| Isocyanat (2) | 35,0 g |
| Katalysator | siehe Tabelle 4 |

| | |
|---|---|
| (1) trifunktionelles Polyetherpolyol, basierend auf Proplenoxidaddukten an Trimethylopropan, Hydroxylgruppengehalt = 11,3 %, Viskosität (20°C) 600 = mPA·s | |
| (2) 4,4-Methylendiphenylisocyanat mit einem NCO-Gehalt von 31,0 %, Viskosität (20°C) = 110 mPa·s | |

**Tabelle 4**

| Kennwerte der PU-Gießharzes | | | | |
|---|---|---|---|---|
| Katalysator | Menge [pphp] | Topfzeit [min] | Temp./Topfzeit [°C] | Entformzeit [min] |
| Mischung ^{K} | 0,25 | 11 | 70 | 60 |
| I (erf.gem.) | 0,4 | 40 | 42 | 90 |
| unkatalysiert | - | > 50 | 36 | 150 |

| | | | | |
|---|---|---|---|---|
| Mischung K : 17,4 Gew.% 2-Methyl-2-azanorbornan, 60 Gew.% β,β'-Dimorpholinodiethylether, 22,6 Gew.% 2-Ethylhexansäure. | | | | |

Die mit den erfindungsgemäßen Katalysatoren hergestellten Polyurethane wiesen keinen Geruch auf. Ein deutlich verminderter Geruch gegenüber den Produkten des Standes der Technik trat auf, wenn die erfindungsgemäßen Katalysatoren mit Amincokatalysatoren eingesetzt wurden. Der dann noch feststellbare Amingeruch stammte von den Cokatalysatoren.

## Patentansprüche

1. Aminocarbonat-Verbindungen der allgemeinen Formel I 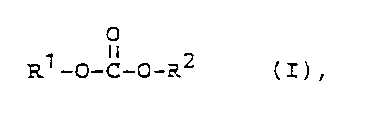 worin R¹ und R², die gleich oder verschieden sind, eine tertiäre Aminogruppe sind, die ein Rest der allgemeinen Formel II 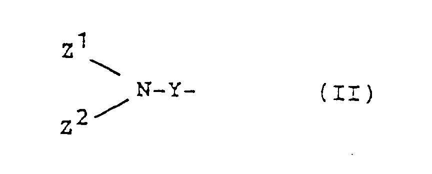 ist bzw. sind, worin Z¹ und Z² zusammen einen Morpholin- oder Piperazin-Rest der allgemeinen Formel III bzw. IV bilden 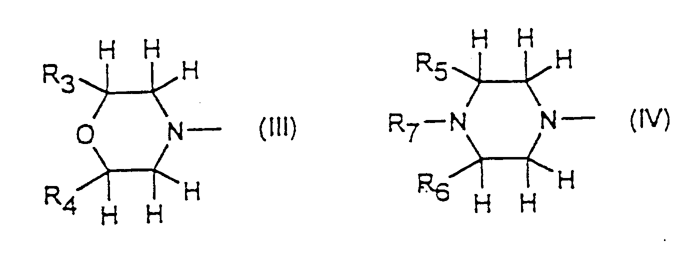 worin die Reste R³ und R⁴ und die Reste R⁵ und R⁶ gleich oder verschieden sind und jeweils Wasserstoff und/oder Alkylreste mit 1 bis 2 Kohlenstoffatomen, und R⁷ Wasserstoff oder einen Alkylrest mit 1 bis 2 Kohlenstoffatomen bedeuten, und
Y ein unverzweigtes oder verzweigtes Alkylen mit 2 bis 10 Kohlenstoffatomen bedeutet.

2. Verbindungen nach Anspruch 1, dadurch gekennzeichnet, daß die Reste R³ bis R⁷ Wasserstoff und/oder Methyl darstellen.

3. Verbindungen nach Anspruch 1, dadurch gekennzeichnet, daß die Reste R³ bis R⁷ Wasserstoff darstellen.

4. Verbindungen nach einem der Ansprüche 1 bis 3, dadurch gekennzeichnet, daß Y ein Alkylen mit 2 bis 4 Kohlenstoffatomen ist.

5. Verbindung nach Anspruch 1, dadurch gekennzeichnet, daß die Aminocarbonat-Verbindung Dimorpholinoethylcarbonat ist.

6. Anwendung einer oder mehrerer Verbindungen nach einem der vorhergehenden Ansprüche als Katalysator(en) zur Herstellung von Polyurethanen und/oder Polyharnstoffen.

7. Anwendung nach Anspruch 6 zusammen mit an sich bekannten Katalysatoren zur Herstellung von Polyurethanen und/oder Polyharnstoffen.

## Claims

1. Amino carbonate compounds corresponding to general formula I 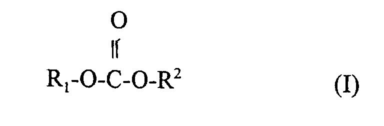 wherein R¹ and R² are the same or different, are a tertiary amino group in which a radical of general formula II 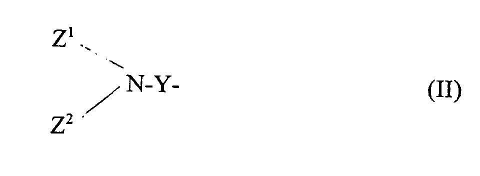 wherein Z¹ and Z² together form a morpholine or piperazine which is or are a group of general formula III and IV 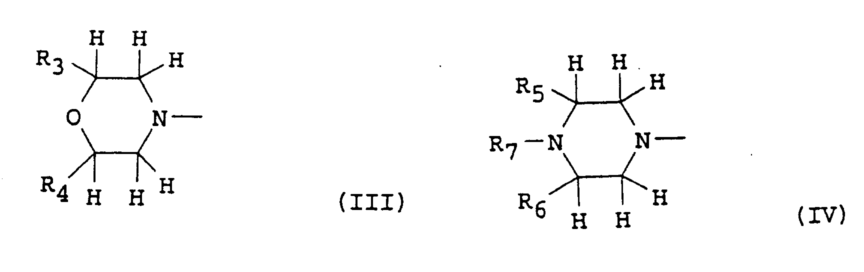 in which the groups R³ and R⁴ and the groups R⁵ and R⁶ are the same or different and respectively denote hydrogen and/or alkyl groups with 1 to 2 carbon atoms and R⁷ denotes hydrogen or an alkyl group with 1 to 2 carbon atoms, and Y denotes an unbranched or branched alkylene with 2 to 10 carbon atoms.

2. Compounds according to claim 1, characterised in that the groups R³ to R⁴ constitute hydrogen and/or methyl.

3. Compounds according to claim 1, characterised in that the groups R³ to R⁷ constitute hydrogen.

4. Compounds according to one of claims 1 to 3, characterised in that Y is an alkaline with 2 to 4 carbon atoms.

5. A compound according to claim 1, characterised in that the aminocarbonate compound is dimorpholinoethyl carbonate.

6. Application of one or more compounds according to one of the preceding claims as a catalyst or as catalysts in the production of polyurethanes and/or polyureas.

7. Application according to claim 6 together with per se known catalysts for the production of polyurethanes and/or polyureas.

## Revendications

1. Composés aminocarbonates de formule générale I 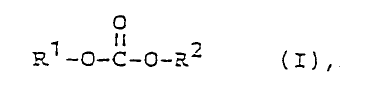 dans laquelle R¹ et R², qui sont identiques ou différents, sont un groupe amino tertiaire qui est un radical de formule générale II 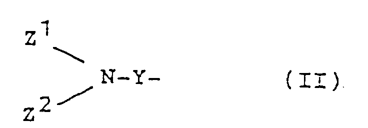 dans laquelle Z¹ et Z² forment ensemble un radical morpholine ou un radical pipérazine respectivement de formule générale III et de formule générale IV 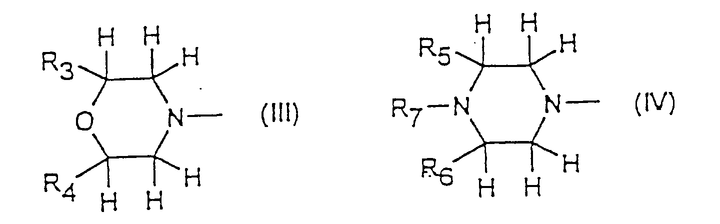 dans lesquelles les radicaux R³ et R⁴ et les radicaux R⁵ et R⁶ sont identiques ou différents et représentent chacun l'hydrogène et/ou des radicaux alkyle ayant de 1 à 2 atomes de carbone, et R⁷ représente l'hydrogène ou un radical alkyle ayant de 1 à 2 atomes de carbone, et Y représente un radical alkylène non ramifié ou ramifié ayant de 2 à 10 atomes de carbone.

2. Composés selon la revendication 1, caractérisés en ce que les radicaux R³ à R⁷ représentent l'hydrogène et/ou le groupe méthyle.

3. Composés selon la revendication 1, caractérisés en ce que les radicaux R³ à R⁷ représentent l'hydrogène.

4. Composés selon l'une des revendications 1 à 3, caractérisés en ce que Y est un radical alkylène ayant de 2 à 4 atomes de carbone.

5. Composé selon la revendication 1, caractérisé en ce que le composé aminocarbonate est le carbonate de dimorpholinoéthyle.

6. Utilisation d'un ou plusieurs composés selon l'une des revendications précédentes en tant que catalyseur(s) pour la préparation de polyuréthannes et/ou de polyurées.

7. Utilisation selon la revendication 6 conjointement avec des catalyseurs connus en soi pour la préparation de polyuréthannes et/ou de polyurées.
